**Europäisches Patentamt**

(19) **European Patent Office**

**Office européen des brevets**

(11) Veröffentlichungsnummer: **0 150 794
B1**

(12) **EUROPÄISCHE PATENTSCHRIFT**

(45) Veröffentlichungstag der Patentschrift:
**13.05.87**

(21) Anmeldenummer: **85100591.8**

(22) Anmeldetag: **21.01.85**

(51) Int. Cl.⁴: **C 07 C 51/09**, C 07 C 53/126,
C 07 C 67/02, C 07 C 69/30,
C 11 C 1/04

(54) Verfahren zur Herstellung von Gemischen aus C6-C10-Fettsäuren.

(30) Priorität: **28.01.84 DE 3403021**

(43) Veröffentlichungstag der Anmeldung:
**07.08.85 Patentblatt 85/32**

(45) Bekanntmachung des Hinweises auf die Patenterteilung:
**13.05.87 Patentblatt 87/20**

(84) Benannte Vertragsstaaten:
**AT BE CH DE FR IT LI LU NL**

(56) Entgegenhaltungen:
**FR - A - 1 206 623**

(73) Patentinhaber: **Henkel Kommanditgesellschaft auf Aktien, Postfach 1100 Henkelstrasse 67,
D-4000 Düsseldorf-Holthausen (DE)**

(72) Erfinder: **Rupilius, Wolfgang, Dr., Am Rittersberg 30,
D-4000 Düsseldorf 13 (DE)**
Erfinder: **Hentschel, Karl, Boschstrasse 40,
D-4000 Düsseldorf 13 (DE)**

## Beschreibung

Ester von natürlich vorkommenden Fettsäuren und kurzkettigen aliphatischen Alkoholen, insbesondere Fettsäuremethylester besitzen grosse technische Bedeutung, vor allem als Ausgangsmaterialien für die Herstellung von Fettalkoholen und deren Folgeprodukten. Sie werden aber auch zur Gewinnung anderer fettchemischer Produkte, beispielsweise von Fettsäurealkanolamiden, Estersulfonaten und Seifen eingesetzt. Für die genannten Zwecke sind vor allem — mit Rücksicht auf die geforderten anwendungstechnischen Eigenschaften der Endprodukte — Alkylester von Fettsäuren mit 12 bis 18 Kohlenstoffatomen, insbesondere in Form von Gemischen, von Interesse. Derartige Fettsäureestergemische werden überwiegend durch katalytische Umesterung von Fetten und Ölen natürlichen Ursprungs, die bekanntlich aus Fettsäuretriglyceriden bestehen, mit den entsprechenden kurzkettigen Alkoholen, vor allem mit Methanol, erhalten.

Die meisten natürlich vorkommenden Fette und Öle ergeben bei der katalytischen Umesterung auf direktem Wege Estergemische, deren Fettsäureanteil aus $C_{12}$-$C_{18}$-Fettsäuren besteht. Setzt man Kokosöl oder Palmkernöl als Ausgangsmaterial für die Umesterung ein, so erhält man Estergemische mit einem beträchtlichen Anteil an Estern kurzkettiger Fettsäuren, deren Anwesenheit zumeist nicht erwünscht ist. Aus diesem Grund trennt man die aus Kokosöl und Palmkernöl erhaltenen Estergemische in der Regel durch Destillation in eine niedrigsiedende Fraktion, die die Ester der $C_6$-$C_{10}$-Fettsäuren (Vorlauffettsäureester) umfasst, und eine höhersiedende Fraktion, die die Ester der $C_{12}$-$C_{18}$-Fettsäuren enthält.

Die Hauptfraktion der Kokosöl- und Palmkernölfettsäureester dient als Ausgangsmaterial für die Herstellung von Fettalkoholen und Fettsäurealkanolamiden. Die abgetrennten Vorlauffettsäureester werden zum Teil über die Hochdruckhydrierung zu $C_6$-$C_{10}$-Fettalkoholen verarbeitet. Der andere Teil wird hydrolytisch zu den freien $C_6$-$C_{10}$-Fettsäuren gespalten, die dann als Vorlauffettsäuren auf den Markt kommen.

Die Überführung der Vorlauffettsäurealkylester in die freien Fettsäuren kann nach bekannten Verfahren durch alkalische Verseifung und anschliessende Zersetzung der Seifen mit Mineralsäuren oder durch Hydrolyse in Gegenwart von sauren Katalysatoren erfolgen.

Die alkalische Verseifung erfordert einen beträchtlichen Aufwand an Chemikalien, da stöchiometrische Mengen Alkalilauge und Mineralsäuren eingesetzt werden müssen. Die entstehenden wässrigen Alkalisalzlösungen führen darüber hinaus zu einer beachtlichen Abwasserbelastung.

Bei der säurekatalysierten Hydrolyse der Fettsäureester handelt es sich um eine Gleichgewichtsreaktion, die nur dann bis zu einem zufriedenstellenden Umsetzungsgrad geführt werden kann, wenn man Wasser in grossem Überschuss einsetzt. Besonders störend ist dabei die Tatsache, dass sich die umzusetzenden Ester in den entstehenden freien Fettsäuren sehr gut lösen, wodurch die Hydrolysegeschwindigkeit stark herabgesetzt wird. Unter den Bedingungen der Betriebspraxis können einwandfreie Vorlauffettsäuren nur dann erhalten werden, wenn die am Ende der Hydrolyse noch vorhandenen Anteile an Alkylestern mit Wasserdampf abgetrieben werden. Im übrigen entstehen hier wie bei der alkalischen Verseifung der Fettsäureester wässrige Alkohollösungen, die durch fraktionierte Destillation aufgearbeitet werden müssen.

Der vorliegenden Erfindung lag demzufolge die Aufgabe zugrunde, ein Verfahren aufzufinden, mit dessen Hilfe aus den Vorlauffettsäureestern kurzkettiger Alkohole in einfacher Weise und unter Vermeidung der Nachteile der bekannten Verfahren die freien Vorlauffettsäuren gewonnen werden können.

Die Lösung dieser Aufgabe geht von der Erkenntnis aus, dass Vorlauffettsäureester kurzkettiger Alkohole in Gegenwart von alkalischen Katalysatoren leicht mit Glycerin zu den entsprechenden Glyceriden umgeestert werden können und dass diese Vorlauffettsäureglyceride im Gemisch mit natürlichen Fetten und Ölen, insbesondere mit Kokosöl und Palmkernöl, unter den Bedingungen der Fettspaltung zu Fettsäuregemischen hydrolysiert werden können, die einen hohen Anteil an $C_6$-$C_{10}$-Fettsäuren besitzen und aus denen die $C_6$-$C_{10}$-Fettsäuren leicht durch Destillation gewonnen werden können.

Gegenstand der Erfindung ist demnach ein Verfahren zur Herstellung von Fettsäuregemischen mit einem hohen Anteil an $C_6$-$C_{10}$-Fettsäuren durch Hydrolyse von Fettsäureglyceriden, dadurch gekennzeichnet, dass man im wesentlichen aus $C_6$-$C_{10}$-Fettsäurealkylestern mit 1 bis 4 Kohlenstoffatomen im Alkylrest bestehende Gemische in Gegenwart von Veresterungskatalysatoren bei 160 bis 250 °C mit Glycerin umestert, die erhaltenen Glyceride im Gemisch mit Kokosöl und/oder Palmkernöl der an sich bekannten Fettspaltung unterwirft und die freigesetzten Fettsäuren isoliert.

Die erfindungsgemäss als Ausgangsmaterial eingesetzten Gemische von Fettsäurealkylestern mit 1 bis 4 Kohlenstoffatomen werden, wie weiter oben bereits erwähnt, als Nebenprodukte bei der Herstellung von Fettalkoholen aus Kokosöl und Palmkernöl erhalten. Der Säureanteil dieser Ester besteht aus Capryl-, Capron- und Caprinsäure. Gegebenenfalls können auch untergeordnete Mengen Laurinsäure vorhanden sein. Die Mengenverhältnisse, in denen die $C_6$-$C_{10}$-Fettsäuren in den Vorlauffettsäureestergemischen vorkommen, sind in Abhängigkeit von der Herkunft der nativen Rohstoffe und von der Durchführung der destillativen Trennung weiten Schwankungen unterworfen. Als typische Beispiele für die Zusammensetzung des Säureanteils von erfindungsgemäss als Ausgangsmaterial zu verwendenden Vorlauffettsäureestern seien die folgenden genannt:

*Vorlauffettsäureesterschnitt aus Kokosöl*

| | |
|---|---|
| Capronsäure | Spuren |
| Caprylsäure | 88,5 Gew.-% |
| Caprinsäure | 11,5 Gew.-% |

*Vorlauffettsäureester aus Palmkernöl*

| | |
|---|---|
| Capronsäure | 1,2 Gew.-% |
| Caprylsäure | 59,7 Gew.-% |

| Caprinsäure | 34,6 Gew.-% |
| Laurinsäure | 4,5 Gew.-% |

Als Alkoholkomponente der Vorlauffettsäurealkylester kommen Ethanol, n-Propanol, iso-Propanol, n-Butanol, sek.-Butanol, tert.-Butanol und vorzugsweise Methanol in Betracht.

Für die Umesterung kann technisches Glycerin verwendet werden; vorzugsweise wird aus der Fettspaltung stammendes Rohglycerin eingesetzt. Das darin jeweils vorhandene Wasser wird zweckmässigerweise durch Erhitzen im Vakuum auf 140-160°C abdestilliert, bevor die Fettsäurealkylester und der Katalysator zugegeben werden.

Bei der Umesterung mit Glycerin ist es zweckmässig, das Molverhältnis von Fettsäurealkylester zu Glycerin nicht kleiner als 1:1 zu wählen. Bevorzugt werden die Fettsäurealkylester mit Glycerin im Molverhältnis von 2:1 bis 3:1 umgeestert.

Als Katalysatoren für die Umesterung mit Glycerin eignen sich praktisch alle Veresterungskatalysatoren, beispielsweise saure Katalysatoren wie Chlorwasserstoff, Schwefelsäure, Phosphorsäure, Bortrifluorid, Aluminiumchlorid, Trichloressigsäure, Alkylschwefelsäuren und organische Sulfonsäuren oder alkalische Veresterungskatalysatoren wie Alkalimetallhydroxide, -carbonate und -acetate, Erdalkalimetalloxide, -hydroxide, -carbonat und -acetat sowie Alkalimetall- und Erdalkalimetallsalze von Fettsäuren mit 6 bis 22 Kohlenstoffatomen. Weiterhin kommen Titanverbindungen, wie organische Titanate, metallisches Zinn und organische Zinnverbindungen, wie Mono- und Dialkylzinnderivate als Umesterungskatalysatoren in Betracht. Vorzugsweise wird die Umesterung mit alkalischen Katalysatoren, insbesondere mit Natriumhydroxid durchgeführt. Die Katalysatoren werden dem Reaktionsgemisch in Mengen von 0,05 bis 1 Gew.-%, bezogen auf die Menge des eingesetzten Fettsäurealkylesters, zugegeben.

Die Umesterung verläuft glatt bei Temperaturen von 160-250°C unter Normaldruck. Bei der praktischen Durchführung des erfindungsgemässen Verfahrens heizt man das Reaktionsgemisch rasch bis zu der Temperatur auf, bei der sich der Reaktionsbeginn durch das Überdestillieren von freigesetztem Monoalkohol bemerkbar macht. Danach wird die Temperatur des Reaktionsgemisches nur noch in dem Masse gesteigert, wie dies für ein möglichst gleichmässiges Abdestillieren des gebildeten Monoalkohols erforderlich ist. Es hat sich als zweckmässig erwiesen, am Ende der Reaktion für kurze Zeit Vakuum (ca. 15-40 mbar) anzulegen, um gegebenenfalls noch vorhandene Spuren von Fettsäurealkylestern aus dem Reaktionsgemisch zu entfernen. Die auf diese Weise erhaltenen Umesterungsprodukte können unmittelbar der nächsten Verfahrensstufe zugeführt werden.

Zur Durchführung der Fettsäurespaltung werden die erhaltenen Vorlauffettsäureglyceride mit Kokosöl und/oder Palmkernöl vermischt, wobei es sich als zweckmässig erwiesen hat, diese Materialien im Gewichtsverhältnis von 1:19 bis 1:3 einzusetzen. Die Gemische aus nativen Ölen und Vorlauffettsäureglyceriden werden sodann mit 100 bis 300 Gew.-% Wasser, vorzugsweise 150 bis 250 Gew.-% Wasser, bezogen auf die Gesamtmenge der umzusetzenden Fettsäureglyceride, ohne Katalysatorzusatz in einen geschlossenen Reaktor unter dem sich einstellenden Druck auf 200-300°C erhitzt und so lange bei diesen Temperaturen gehalten, bis der gewünschte Spaltgrad erreicht ist. Überraschenderweise hat es sich gezeigt, dass die Spaltung von Kokosöl und Palmkernöl durch die Beimischung von Vorlauffettsäureglyceriden erheblich beschleunigt wird. So wurde beispielsweise beobachtet, dass mit reinem Kokosöl bei 225°C das Spaltgleichgewicht mit ca. 95% freien Fettsäuren erst nach 240 Minuten erreicht wurde. Wenn ein Gemisch aus 90 Gewichtsteilen Kokosöl und 10 Gewichtsteilen Vorlauffettsäureglycerid eingesetzt wurde, so konnte das Spaltgleichgewicht bereits nach 180 Minuten erreicht werden.

Aus dem bei der Druckspaltung erhaltenen Reaktionsgemisch können die freigesetzten Fettsäuren ohne Schwierigkeiten als organische Phase abgetrennt und anschliessend durch Waschen gereinigt werden.

In den auf diese Weise erhaltenen Fettsäuren sind die $C_6$-$C_{10}$-Fettsäuren je nach dem vorausgegangenen Zusatz an Vorlauffettsäureglyceriden mehr oder weniger stark angereichert. Im allgemeinen wird man bei der Fettsäurespaltung von Gemischen aus Vorlauffettsäureglyceriden und Kokosöl oder Palmkernöl die Mischungsverhältnisse so wählen, dass Fettsäuregemische entstehen, die 10 bis 40 Gew.-% $C_6$-$C_{10}$-Fettsäuren und im einzelnen

    0-10 Gew.-% Capronsäure
    5-35 Gew.-% Caprylsäure und
    5-35 Gew.-% Caprinsäure

enthalten.

Aus den erhaltenen Fettsäuregemischen mit einem hohen Gehalt an $C_6$-$C_{10}$-Fettsäuren können die Vorlauffettsäuren in einfacher Weise durch Destillation im Vakuum abgetrennt werden. Die auf diese Weise gewonnenen Vorlauffettsäurefraktionen finden hauptsächlich als Ausgangsmaterial für die Herstellung von Esterschmiermitteln Verwendung.

*Beispiel*

*A) Umesterung mit Glycerin*

Als Ausgangsmaterial diente eine aus einem Kokosfettsäuremethylestergemisch abgetrennte Vorlauffraktion, die ca. 88,5 Gew.-% Caprylsäuremethylester, 11,5 Gew.-% Caprinsäuremethylester und Spuren Capronsäuremethylester enthielt. Dieses Material hatte eine Säurezahl von 0,2 und eine Verseifungszahl von 348.

In einem Rührbehälter mit Destillationsaufsatz wurden 1,44 kg Rohglycerin (12,5 Mol) aus der Fettspaltung (ca. 80 Gew.-% Glycerin) unter Rühren und Erhitzen auf 150°C im Wasserstrahlvakuum entwässert. Danach wurden 4,83 kg (30 Mol) Ausgangsmaterial und 10 g Natriumhydroxid (als 50%ige Lösung; 0,1 Gew.-% NaOH, bezogen auf Fettsäuremethylester) zugegeben. Unter Rühren bei Normaldruck wurde nun die Temperatur des Gemisches langsam gesteigert. Bei 160°C machte sich das Einsetzen der Umesterungsreaktion dadurch bemerkbar, dass Methanol überdestillierte. Im Verlauf von 4 Stunden wurde die Temperatur des Gemisches auf 220°C erhöht. Zum Schluss wurde kurzzeitig Vakuum (ca. 20 mbar) angelegt, um Spuren von nicht umgesetztem Methylester zu entfernen. Die Destillation wurde abgebrochen, sobald Fettsäuremonoglycerid (erkenntlich an der höheren Viskosität) im Destillat erschien.

Das erhaltene Vorlauffettsäureglyceridgemisch hatte eine Säurezahl von 0,6 und eine Verseifungszahl von 304,9.

*B) Fettspaltung*

1) In einem 5 l fassenden Autoklaven mit Rührwerk wurden 2 kg Wasser auf 225°C erhitzt, bevor über eine Förderpumpe 1 kg eines Gemisches auf 95 Gew.-% Kokosöl (Zusammensetzung des Fettsäureanteils in Gewichtsprozent: 0,7 $C_6$; 8,0 $C_8$; 6,2 $C_{10}$; 48,1 $C_{12}$; 17,6 $C_{14}$; 8,6 $C_{16}$; 10,8 $C_{18}$; Säurezahl 5,6; Verseifungszahl 254,6) und 5 Gew.-% des oben beschriebenen Vorlauffettsäureglycerids in den Autoklaven eingespeist wurde. Das entstandene Gemisch wurde weiterhin auf einer Temperatur von 225°C gehalten, während gleichzeitig über ein Ventil in regelmässigen Abständen Proben für die Bestimmung der vorherrschenden Säurezahlen und Verseifungszahlen entnommen wurden. Anhand der analytischen Daten wurde festgestellt, dass nach 200 Minuten ein Spaltgrad von 95% erreicht war.

Das aus dem Reaktionsgemisch isolierte Fettsäuregemisch hatte nach gaschromatographischer Analyse folgende Zusammensetzung (in Gewichtsprozent): 0,6 $C_6$; 11,9 $C_8$; 6,6 $C_{10}$; 45,9 $C_{12}$; 16,5 $C_{14}$; 8,3 $C_{16}$; 10,2 $C_{18}$.

2) In Analogie zu B1) wurde ein Gemisch aus 90 Gew.-% Kokosöl und 10 Gew.-% Vorlauffettsäureglycerid der Fettspaltung bei 225°C unterworfen. Dabei wurde ein Spaltgrad von 95% nach 165 Minuten erhalten.

Das aus dem Reaktionsgemisch gewonnene Fettsäuregemisch hatte nach gaschromatographischer Analyse die Zusammensetzung (in Gewichtsprozent): 0,6 $C_6$; 16,2 $C_8$; 6,7 $C_{10}$; 43,5 $C_{12}$; 15,6 $C_{14}$; 7,7 $C_{16}$; 9,7 $C_{18}$.

3) In Analogie zu B1) wurde ein Gemisch aus 75 Gew.-% Kokosöl und 25 Gew.-% Vorlauffettsäureglycerid bei 225°C der Fettspaltung unterworfen. Ein Spaltgrad von 95% wurde dabei nach 140 Minuten erreicht.

Das aus dem Reaktionsgemisch gewonnene Fettsäuregemisch hatte nach gaschromatographischer Analyse die Zusammensetzung (in Gewichtsprozent): 0,4 $C_6$; 28,1 $C_8$; 7,7 $C_{10}$; 36,2 $C_{12}$; 13,2 $C_{14}$; 6,3 $C_{16}$; 8,1 $C_{18}$.

4) Zu Vergleichszwecken wurde Kokosöl ohne Zusatz von Vorlauffettsäureglycerid unter den in B1) wiedergegebenen Bedingungen gespalten. Dabei wurde ein Spaltgrad von 95% erst nach 240 Minuten erreicht.

**Patentansprüche**

1. Verfahren zur Herstellung von Fettsäuregemischen mit einem hohen Anteil an $C_6$-$C_{10}$-Fettsäuren durch Hydrolyse von Fettsäureglyceriden, dadurch gekennzeichnet, dass man im wesentlichen aus $C_6$-$C_{10}$-Fettsäurealkylestern mit 1 bis 4 Kohlenstoffatomen im Alkylrest bestehende Gemische in Gegenwart von Umesterungskatalysatoren bei 160 bis 250°C mit Glycerin umestert, die erhaltenen Glyceride im Gemisch mit Kokosöl und/oder Palmkernöl der an sich bekannten Fettspaltung unterwirft und die freigesetzten Fettsäuren isoliert.

2. Verfahren nach Anspruch 1, dadurch gekennzeichnet, dass man im wesentlichen aus $C_6$-$C_{10}$-Fettsäuremethylestern bestehende Gemische mit Glycerin umestert.

3. Verfahren nach den Ansprüchen 1 und 2, dadurch gekennzeichnet, dass man die Fettsäurealkylester mit Glycerin im Molverhältnis von 2:1 bis 3:1 umestert.

4. Verfahren nach den Ansprüchen 1 bis 3, dadurch gekennzeichnet, dass man die Umesterung mit Glycerin in Gegenwart von alkalischen Katalysatoren durchführt.

5. Verfahren nach den Ansprüchen 1 bis 4, dadurch gekennzeichnet, dass man die Umesterung mit Glycerin in Gegenwart von Natriumhydroxid durchführt.

6. Verfahren nach den Ansprüchen 1 bis 5, dadurch gekennzeichnet, dass man die erhaltenen Glyceride im Gemisch mit Fetten und/oder Ölen im Gewichtsverhältnis von 1:19 bis 1:3 der Fettspaltung unterwirft.

**Claims**

1. A process for the production of fatty acid mixtures containing a high percentage of $C_6$-$C_{10}$ fatty acids by hydrolysis of fatty acid glycerides, characterized in that mixtures consisting essentially of $C_6$-$C_{10}$ fatty acid alkyl esters containing from 1 to 4 C-atoms in the alkyl radical are transesterified with glycerol at 160 to 250°C in the presence of transesterification catalysts, the glycerides obtained are subjected to lipolysis known per se in admixture with coconut oil and/or palm kernel oil and the fatty acids released are isolated.

2. A process as claimed in Claim 1, characterized in that mixtures consisting essentially of $C_6$-$C_{10}$ fatty acid methyl esters are transesterified with glycerol.

3. A process as claimed in Claims 1 and 2, characterized in that the fatty acid alkyl esters are transesterified with glycerol in a molar ratio of from 2:1 to 3:1.

4. A process as claimed in Claims 1 to 3, characterized in that the transesterification with glycerol is carried out in the presence of alkaline catalysts.

5. A process as claimed in Claims 1 to 4, characterized in that the transesterification with glycerol is carried out in the presente of sodium hydroxide.

6. A process as claimed in Claims 1 to 5, characterized in that the glycerides obtained are subjected to the lipolysis in admixture with fats and/or oils in a ratio by weight of from 1:19 to 1:3.

## Revendications

1. Procédé de fabrication de mélanges d'acides gras comportant une fraction importante d'acides gras $C_6$-$C_{10}$ par hydrolyse de glycérides d'acides gras, caractérisé en ce qu'à des températures de 160 à 250°C on alcoolyse avec du glycérol des mélanges composés essentiellement d'esters alkyliques d'acides gras $C_6$-$C_{10}$, dont le radical alkyle comporte 1 à 4 atomes de carbone, en présence de catalyseurs d'alcoolyse, en ce qu'on soumet les glycérides obtenus mélangés à de l'huile de coco ou de palmiste à la dissociation des lipides connue en soi et en ce qu'on isole les acides gras libérés.

2. Procédé selon la revendication 1, caractérisé en ce qu'on alcoolyse des mélanges composés essentiellement d'esters méthyliques d'acides gras $C_6$-$C_{10}$ avec du glycérol.

3. Procédé selon les revendications 1 et 2, caractérisé en ce qu'on alcoolyse les esters alkyliques d'acides gras avec du glycérol selon le rapport molaire de 2:1 à 3:1.

4. Procédé selon les revendications 1 à 3, caractérisé en ce qu'on réalise l'alcoolyse avec du glycérol en présence de catalyseurs alcalins.

5. Procédé selon les revendications 1 à 4, caractérisé en ce qu'on réalise l'alcoolyse avec du glycérol en présence d'hydroxyde de sodium.

6. Procédé selon les revendications 1 à 5, caractérisé en ce qu'on fait subir une dissociation aux glycérides obtenus mélangés à des graisses et/ou des huiles selon le rapport pondéral de 1:19 à 1:3.